# DEMANDE DE BREVET EUROPEEN

(11) **EP 4 275 583 A2**
(43) Date de publication de la demande: **15.11.2023**
(21) Numéro de dépôt: 23192965.4
(22) Date de dépôt: 29.10.2019
(51) Int. Cl.: A61B 1/00

(54) **KIT DE PRISE DE PHOTOS DENTAIRES ARTICULE**

(30) Priorité: 30.10.2018 FR 1860037
(62) Demande divisionnaire de: 19790571.4
(71) Demandeur: Dental Monitoring, 75008 Paris (FR)
(72) Inventeur: SALAH, Philippe, Paris (FR); PELLISSARD, Thomas, Paris (FR); LANCON, Cédric, Villers Farlay (FR); DEBRAUX, Laurent, Paris (FR)
(74) Mandataire: Cabinet Nony

(57) **Abrégé**

Kit de prise de photos dentaires comportant :
- un support comportant une partie d'attache de support ;
- un écarteur dentaire définissant une ouverture d'écarteur et comportant une partie d'attache d'écarteur fixée de manière amovible, dans un position montée de l'écarteur sur le support, sur la partie d'attache de support ; et
- un appareil photo fixé, de manière amovible, sur le support dans une position de service dans laquelle l'appareil photo est orienté pour recevoir une image au moins partielle de l'ouverture d'écarteur,
caractérisé en ce que l'appareil photo, d'axe optique O, est intégré dans un téléphone portable et le téléphone portable est monté mobile sur le support dans un plan perpendiculaire audit axe optique.

## Description

### Domaine technique

La présente invention concerne un dispositif de prise de photos dentaires, en particulier pour la mise en oeuvre d'un procédé tel que décrit dans la demande internationale PCT/EP2015/074896.

### Etat de la technique

PCT/EP2015/074897 décrit un kit d'acquisition permettant d'acquérir des photos des dents d'un patient. Ce kit d'acquisition comporte :
- un écarteur dentaire destiné à être placé dans la bouche du patient ;
- un téléphone portable pourvu d'un appareil photo.

L'acquisition, par le patient, de photos représentant certaines régions de la bouche, et en particulier le palais, reste cependant difficile. La recherche des conditions d'acquisition des photos est également complexe.

US 2018/0228359 décrit un dispositif d'acquisition d'images dentaires comportant un écarteur dentaire et un téléphone portable.

Un objectif de la présente invention est de répondre, au moins partiellement, à ces problèmes.

### Résumé de l'invention

L'invention propose un kit de prise de photos dentaires, le kit comportant :
- un support comportant une partie d'attache de support ;
- un écarteur dentaire définissant une ouverture d'écarteur d'axe X et comportant une partie d'attache d'écarteur fixée de manière amovible, dans une position montée de l'écarteur sur le support, sur la partie d'attache de support ; et
- un appareil photo, de préférence intégré dans un téléphone portable, fixé, de manière rigide ou de préférence amovible, sur le support dans une position de service dans laquelle l'appareil photo est orienté pour recevoir une image au moins partielle de l'ouverture d'écarteur.

**Suivant un premier aspect principal de l'invention,** la partie d'attache d'écarteur est articulée sur la partie d'attache de support.

Comme cela apparaîtra plus clairement dans la suite de la description, le support permet avantageusement de positionner l'appareil photo par rapport à l'écarteur dentaire, ce qui permet d'accélérer le réglage de l'appareil photo lors de la prise des photos, tandis que l'articulation de la partie d'attache d'écarteur par rapport à la partie d'attache de support, et de préférence l'articulation de l'écarteur par rapport au support, permet de facilement modifier les régions de la bouche observées par l'appareil photo à travers l'ouverture d' écarteur.

L'acquisition des photos par le patient en est facilitée.

Une articulation est une liaison autorisant une rotation autour d'au moins un axe de rotation.

Dans un mode de réalisation préféré, la partie d'attache d'écarteur est montée pivotante sur la partie d'attache de support. L'articulation est donc une liaison pivot, de préférence autour d'un axe de pivotement, de préférence horizontal dans la position de service. Elle n'autorise alors qu'une rotation autour de cet axe. De préférence, le pivotement autorise, au moins dans des positions extrêmes de pivotement basse et haute, des prises de photos du palais et de la langue, respectivement.

L'axe de pivotement peut être fixe par rapport au support. De préférence, l'axe de pivotement est mobile en translation par rapport au support, suivant une direction différente de celle de l'axe de pivotement, de préférence suivant une direction perpendiculaire à l'axe de pivotement, comme suivant le deuxième aspect principal de l'invention décrit ci-après.

La partie d'attache de support peut être montée à rotation, autour de l'axe de pivotement, sur un corps du support. La partie d'attache d'écarteur peut être montée à rotation, autour de l'axe de pivotement, sur la partie d'attache de support.

Dans le mode de réalisation préféré de l'invention, dans la position montée de l'écarteur, la partie d'attache d'écarteur est fixée magnétiquement sur la partie d'attache du support.

De préférence, l'écarteur est fixé, de manière amovible, de préférence magnétiquement, par exactement deux parties d'attache d'écarteur coopérant avec deux parties d'attache de support respectives. De préférence, chaque partie d'attache de support étant montée à rotation autour d'un pivot respectif, les deux pivots étant de préférence d'axe Ya et Yb. De préférence, les deux parties d'attache d'écarteur sont des aimants, de préférence noyées dans la masse de l'écarteur.

De préférence, chaque partie d'attache de support est un aimant orienté pour attirer un insert métallique ou un aimant d'une attache d'écarteur correspondante. De préférence, la partie d'attache d'écarteur est un insert métallique et la partie d'attache de support est un aimant. Avantageusement, des essais ont montré que la partie d'attache d'écarteur résiste ainsi mieux à l'étuvage.

**Suivant un deuxième aspect principal de l'invention,** dans la position montée de l'écarteur sur le support, au moins une partie d'attache d'écarteur, de préférence chaque partie d'attache d'écarteur est mobile en translation, suivant un axe de translation respectif, par rapport au support.

Autrement dit, la partie d'attache d'écarteur peut être écartée du support suivant l'axe de translation.

Comme cela apparaîtra plus clairement dans la suite de la description, ladite translation facilite avantageusement la prise de photos ou de films de parties d'arcade dentaire qui ne sont pas dans l'axe longitudinal du support.

De préférence, l'axe de translation est sensiblement parallèle à l'axe X de l'ouverture d'écarteur et/ou sensiblement perpendiculaire à un axe de pivotement de la partie d'attache d'écarteur par rapport à une partie d'attache de support à laquelle ladite partie d'attache d'écarteur est fixée.

De préférence, deux plans perpendiculaires à l'axe de translation et à l'axe de l'ouverture d'écarteur X, respectivement forment toujours entre eux un angle inférieur à 30°, de préférence inférieur à 20°, et/ou, de préférence supérieur à 5°, de préférence supérieur à 10°.

De préférence, deux plans perpendiculaires à l'axe de translation et à l'axe de pivotement d'une partie d'attache d'écarteur, respectivement, forment toujours entre eux un angle supérieur à 80° et inférieur à 110°, de préférence un angle de 90°.

De préférence, lorsque, dans la position montée de l'écarteur, la position et/ou la géométrie de l'écarteur évolue, l'axe de translation d'une partie d'attache d'écarteur s'étend toujours dans un même plan. De préférence, ce plan forme, avec le plan longitudinal médian du support, un angle inférieur à 30°, de préférence inférieur à 20°, et/ou, de préférence supérieur à 5°, de préférence supérieur à 10°.

De préférence, l'amplitude de la translation est limitée. De préférence, l'amplitude maximale est inférieure à 2 cm, de préférence inférieure à 1 cm et/ou supérieure à 0,5 cm.

Dans un mode de réalisation, au moins une des parties d'attache de support et d'attache d'écarteur, de préférence la partie d'attache de support est télescopique suivant l'axe de translation.

**Suivant un troisième aspect principal de l'invention,** l'appareil photo est intégré dans un téléphone portable et le support comporte
- un corps, de préférence sous la forme d'un boîtier, et
- une porte montée pivotante sur le corps autour d'un pivot de porte d'axe P, de préférence sensiblement horizontal dans la position de service, entre une position fermée dans laquelle elle maintient le téléphone portable serré contre une face de réception du corps, et une position ouverte dans laquelle elle autorise une extraction du téléphone portable,
le pivot de porte étant monté coulissant par rapport au corps, par exemple dans un rail du corps, selon un axe de coulissement Z qui n'est pas parallèle à l'axe P, de préférence selon un axe de coulissement coplanaire à l'axe P et de préférence sensiblement perpendiculaire à l'axe P.

De préférence, deux plans perpendiculaires aux axes P et Z, respectivement forment toujours entre eux un angle supérieur à 80° et inférieur à 110°, de préférence un angle de 90°.

De préférence, l'axe de coulissement Z forme, avec le plan général de la face de réception du corps, un angle supérieur à 80° et inférieur à 110°, de préférence un angle de 90°.

Comme cela apparaîtra plus clairement dans la suite de la description, la porte peut ainsi s'écarter de la face de réception, tout en restant, dans la position fermée, sensiblement parallèle à la face de réception. Le support peut ainsi avantageusement s'adapter à des téléphones mobiles présentant différentes épaisseurs.

De préférence, le support comporte un organe de rappel, de préférence un ressort ou un aimant, disposé de manière à s'opposer, de préférence élastiquement, à un écartement du pivot de porte à distance du plan de la face de réception, en particulier lorsque la porte est maintenue parallèle à la face de réception. L'action de l'organe de rappel participe avantageusement à la fixation du téléphone portable.

De préférence, dans la position ouverte de la porte, le téléphone portable repose une face de réception du support, de préférence plane, et le support comporte seulement des première et deuxième butées,
- la première butée, de préférence une butée latérale, limitant, dans la position ouverte de la porte, le glissement du téléphone portable, sur la face de réception, suivant une première direction et dans un seul sens, et
- la deuxième butée, de préférence une butée inférieure, limitant, dans la position ouverte de la porte, le glissement du téléphone portable, sur la face de réception, suivant une deuxième direction et dans un seul sens.

Avantageusement, le support est ainsi adapté pour recevoir des téléphones mobiles de différentes longueurs et/ou largeurs.

De préférence, les première et deuxième directions, différentes, forment un angle de préférence supérieur à 60° et/ou inférieur à 120°, de préférence d'environ 90°.

De préférence, le support présente la forme d'un boîtier ne débouchant vers l'extérieur sensiblement que par une ouverture proximale, obturée par le téléphone portable dans la position fermée, et une ouverture distale, débouchant dans l'ouverture d'écarteur.

**Suivant un quatrième aspect principal de l'invention,** l'appareil photo, d'axe optique O, est intégré dans un téléphone portable et le téléphone portable est monté mobile sur le support dans un plan perpendiculaire audit axe optique.

Comme cela apparaîtra plus clairement dans la suite de la description, la mobilité du téléphone permet un centrage très précis de l'objectif sur l'axe longitudinal du support.

De préférence, le support comporte un boîtier et un cadre de fixation du téléphone portable, le cadre étant guidé en translation sur le boîtier.

De préférence, le cadre comporte des marques d'identification de la position du cadre par rapport au boîtier et le boîtier comporte un repère en face duquel chaque marque d'identification peut être positionnée. De préférence, les marques d'identification correspondent à des modèles de téléphone portable. Autrement dit, pour positionner correctement un téléphone portable sur le support, il suffit de positionner la marque d'identification correspondant à ce téléphone portable face au repère.

De manière équivalente, le repère peut être sur le cadre et les marques d'identification sur le boitier.

De préférence, la mobilité du téléphone portable est indexée, c'est-à-dire se fait par saut entre des position stables prédéterminées. De manière conventionnelle, un cliquet élastique du cadre peut par exemple coopérer avec des trous correspondant sur le boîtier.

Dans un mode de réalisation, le boîtier comporte un moyen, sélectivement activable et désactivable par l'opérateur, pour bloquer le téléphone portable en position.

De préférence, la mobilité du téléphone portable est limitée à une translation suivant un axe ou à deux translations suivant deux axes, de préférence sensiblement perpendiculaires l'un à l'autre.

Bien entendu, les caractéristiques, optionnelles ou non, des différents aspects principaux de l'invention, peuvent être combinées.

En particulier, au moins une, de préférence chaque attache de l'écarteur sur le support autorise une rotation, de préférence un pivotement autour de l'axe de pivotement, et un écartement, suivant l'axe de translation, de la partie d'attache l'écarteur par rapport au support.

Un kit selon l'invention présente de préférence une ou plusieurs des caractéristiques optionnelles suivantes :
- le support porte un miroir fixé et orienté de manière que l'appareil photo du téléphone portable reçoive une image composée comportant une image directe de l'écarteur et une image de l'ouverture d'écarteur réfléchie par le miroir ;
- le support porte une mire colorimétrique et/ou une mire de translucidité ;
- lorsque l'appareil photo est intégré dans le support, il comporte des moyens de communication sans fil, de préférence des moyens de communication par téléphonie, ce qui facilite la mise en oeuvre des procédés décrits dans PCT/EP2015/074897.

### Définitions

Par « patient », on entend toute personne pour laquelle un kit selon l'invention peut être mis en oeuvre, que cette personne soit malade ou non, ou que cette personne soit en cours de traitement ou non. Un kit selon l'invention peut être utilisé pour un autre animal qu'un être humain.

On appelle classiquement "téléphone portable", un appareil de type iPhone^{®}. Un tel appareil pèse typiquement moins de 500 g, est doté d'un appareil photo lui permettant de prendre des films ou des photos, est capable d'échanger des données avec un autre appareil éloigné de plus de 500 km du téléphone portable, et est capable d'afficher lesdites données, et notamment lesdites photos. Un « film » est considéré comme un ensemble de photos.

« Fixer » deux pièces signifie établir un lien physique entre ces deux pièces, de sorte que tout déplacement de l'une de ces pièces finit par entrainer celui de l'autre pièce. Une pièce montée en translation, à rotation ou à rotule sur une autre pièce est fixée à cette autre pièce. Une fixation peut être « définitive » ou « de manière amovible », selon que les deux pièces sont adaptées ou non à une désolidarisation, de préférence à la main, par un opérateur. Une fixation peut être rigide ou non, selon qu'elle autorise ou non un déplacement relatif des deux pièces fixées l'une à l'autre.

Une fixation magnétique utilise l'attraction d'un aimant et d'une pièce métallique, ou l'attraction entre deux aimants.

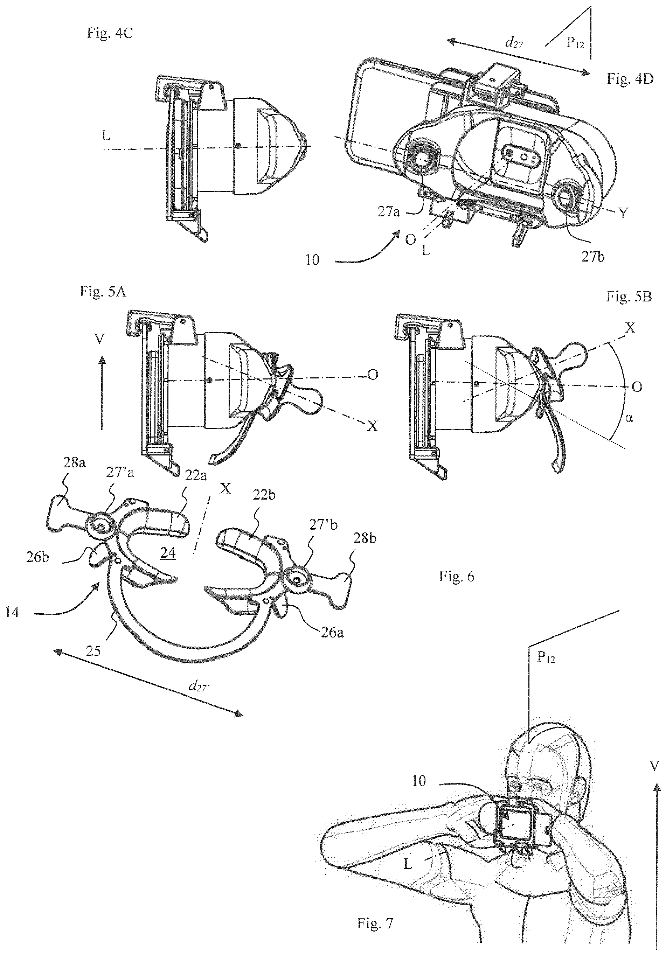

Sauf indications contraires, les qualificatifs utilisés pour définir des positions dans l'espace comme « horizontal », « vertical », « bas », « haut », « droite » ou « gauche » sont définies, à des fins de clarté, en référence à une position de service observée par un patient, ayant la tête droite et ayant disposé ses lèvres sur l'écarteur du kit dans sa position montée, comme représenté sur la figure 7, où V représente la direction verticale, orientée vers le haut.

Dans la position de service, le plan longitudinal médian P₁₂ est le plan vertical qui passe par l'axe longitudinal du support. L'axe longitudinal L du support est l'axe qui s'étend sensiblement horizontalement dans la position de service et qui passe par le centre de la chambre définie par le support et s'étendant entre l'écarteur et le téléphone portable.

"Comprendre", "comporter" et "présenter" doivent être interprétés de manière large et non limitative, sauf indication contraire.

### Brève description des figures

D'autres caractéristiques et avantages de l'invention apparaîtront encore à la lecture de la description détaillée qui va suivre et à l'examen du dessin annexé dans lequel :
- les figures 1A-1B représentent, en perspective et vu de côté, le support d'un kit selon l'invention, en position ouverte de la porte ;
- les figures 2A-2B représentent, en perspective, le support représenté sur la figure 1 et le téléphone portable d'un kit selon l'invention, en position ouverte de la porte, le téléphone portable étant positionné de manière à pouvoir prendre des photos de l'ouverture distale du boîtier ;
- les figures 3A-3B représentent, en perspective et en coupe longitudinale, le support et le téléphone portable représentés sur la figure 2, en position fermée de la porte, le crochet de verrouillage étant en position déverrouillée ;
- les figures 4A-4D représentent, en perspective et vu de côté (figure 4C), le support et le téléphone portable représentés sur la figure 3, en position fermée de la porte, le crochet de verrouillage étant en position verrouillée ;
- les figures 5A-5B représentent, vu de côté, un kit selon l'invention, dans la position de service, dans les positions extrêmes de pivotement du support par rapport à l'écarteur (positions basse et haute, respectivement), seule une moitié de l'écarteur étant représentée ;
- la figure 6 représente, en perspective, un exemple préféré d'écarteur d'un kit selon l'invention ;
- la figure 7 illustre l'utilisation d'un kit selon l'invention par un patient ;
- la figure 8 est un exemple de photo prise lorsque le support comporte des miroirs ;
- les figures 9A-9C illustrent la fixation d'un téléphone portable sur un support, dans un autre mode de réalisation préféré de l'invention ;
- les figures 10A-10F illustrent une mobilité verticale du téléphone portable sur le support ;
- les figures 11A-11C illustrent la translation des parties d'attache d'écarteur par rapport au support.

Dans les différentes figures, les organes identiques ou analogues ont été désignés avec des références identiques.

### Description détaillée

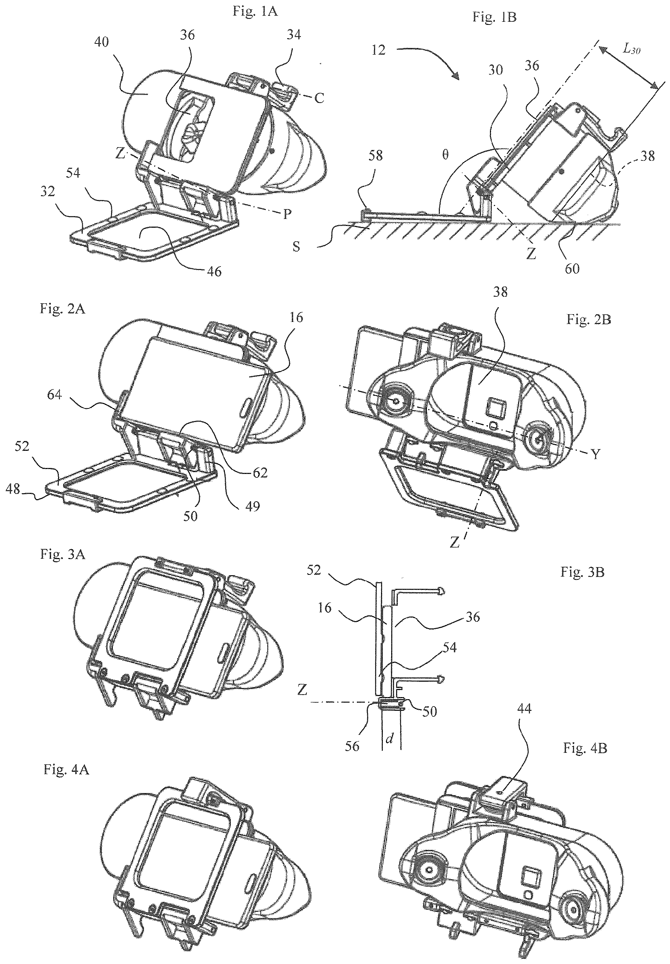

Le kit de prise de photos 10 représenté sur la figure 1 comporte un support 12, un écarteur dentaire 14, et un téléphone portable 16.

### Ecarteur

L'écarteur 14 peut présenter les caractéristiques des écarteurs conventionnels.

Comme représenté sur la figure 6, il comporte de préférence un rebord, de préférence en deux parties 22a et 22b, s'étendant autour d'une ouverture d'écarteur 24 d'axe X et agencé de manière que les lèvres du patient puissent y reposer en laissant apparaître les dents du patient à travers ladite ouverture d'écarteur. Les lèvres du patient sont alors retroussées autour des dents du patient.

Les deux parties du rebord s'étendent de préférence sensiblement symétriquement par rapport à un plan médian passant par l'axe X, de préférence vertical, et sont de préférence reliées entre elles par une poignée 25. La poignée 25 facilite la manipulation, mais aussi autorise un rapprochement élastique des deux parties du rebord l'une vers l'autre. La mise en position des lèvres sur le rebord en est facilitée.

De préférence, l'écarteur comporte des oreilles d'écartement des joues 26a et 26b afin que l'appareil photo puisse acquérir, à travers l'ouverture d'écarteur, des photos de faces vestibulaires de dents disposée au fond de la bouche, comme des molaires. Cette caractéristique est particulièrement avantageuse pour la mise en oeuvre des procédés décrits dans PCT/EP2015/074897.

Dans le mode de réalisation représenté, l'écarteur 14 comporte également des pattes gauche 28a et droite 28b, sensiblement perpendiculaires à l'axe X, facilitant sa manipulation.

L'écarteur 14 est de préférence en un matériau biocompatible, par exemple en matière plastique.

L'écarteur est monté de manière amovible sur le support, c'est-à-dire peut être disposé dans des positions montée et démontée dans lesquelles il est fixé sur le support ou détaché du support, respectivement.

L'écarteur 14 peut être fixé sur le support par une ou plusieurs attaches 27a-27a' et 27b et 27b', de préférence par deux attaches. Une attache assure la fixation de l'écarteur sur le support par coopération d'une partie d'attache de support 27a-27b (Fig. 5B) et d'une partie d'attache d'écarteur (27a'-27b').

Dans un mode de réalisation préféré, l'écarteur est articulé sur le support 12, par tout moyen.

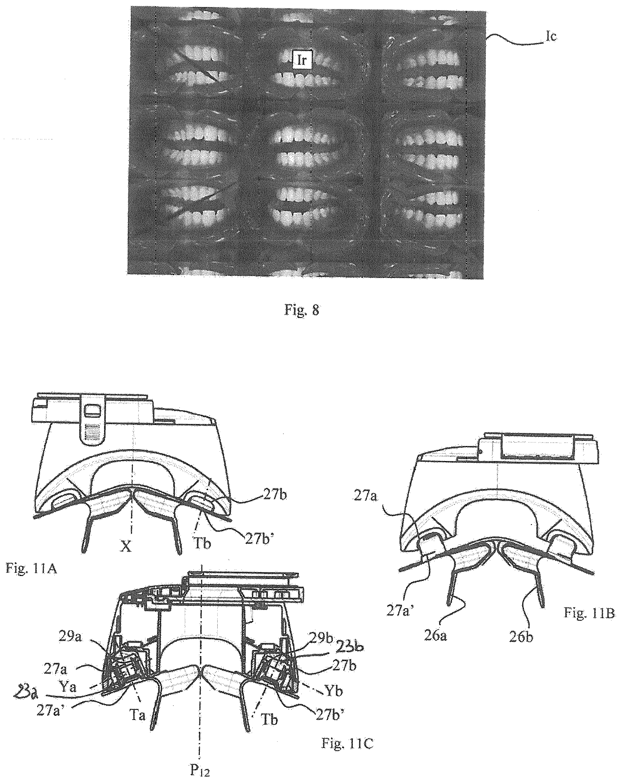

De préférence, l'écarteur 14 est monté à rotation sur le support. De préférence, comme représenté, le support comporte deux parties d'attache de support 27a et 27b disposés symétriquement de part et d'autre du plan longitudinal médian P₁₂ du support. Chaque partie d'attache de support est montée pivotante autour d'un axe de pivotement Ya et Yb respectif (Fig. 11C), de préférence exclusivement autour d'un axe de pivotement Ya et Yb respectif, de préférence sensiblement horizontal, de préférence formant un angle supérieur à 60° et inférieur à 80° avec le plan général de l'ouverture d'écarteur 24. A cet effet, des pivots 23a et 23b, d'axes Ya et Yb, sont solidaires du boîtier, et les parties d'attaches sont montées pivotantes autour de ces pivots 23a et 23b.

Les pivots sont dits « solidaires du boîtier » lorsqu'ils sont fixés rigidement au boîtier ou de manière à n'autoriser qu'un déplacement guidé par rapport au boîtier, par exemple par pivotement autour d'un axe, ou par rotation, ou par translation.

Ces pivotements autorisent un pivotement de l'écarteur autour d'un axe Y (Fig. 4D), ce qui facilite considérablement la prise de photos du palais et de la langue, en guidant les mouvements de l'opérateur lors de la prise des photos.

De préférence, l'amplitude maximale de pivotement, représentée par l'angle α sur la figure 5B, est supérieure à 5°, voire supérieure à 10°, et/ou inférieure à 30°, de préférence inférieure à 20° ou à 15°.

De préférence, le support comporte des butées configurées pour limiter l'amplitude du pivotement. De préférence, les butées sont configurées de manière que les positions de pivotement extrêmes autorisent l'observation de toutes les dents de la bouche du patient. L'écarteur est amovible, c'est-à-dire qu'il peut être monté et démonté du support par l'opérateur. Avantageusement, le même support peut donc servir pour plusieurs écarteurs, et en particulier pour plusieurs écarteurs de tailles différentes.

De préférence, chaque partie d'attache de support est fixée magnétiquement à une partie d'attache d'écarteur correspondante.

Dans un mode de réalisation, un aimant de l'écarteur est disposé de part et d'autre du plan médian de l'écarteur et coopère avec un insert métallique respectif du support, ce qui assure un maintien en position efficace. De préférence cependant, la configuration est inversée, les aimants étant sur le support et les inserts métalliques étant dans l'écarteur. Avantageusement, les inserts métalliques de l'écarteur n'attirent donc pas les objets métalliques lorsque l'écarteur n'est pas fixé sur le support.

Dans un mode de réalisation, les inserts métalliques sont remplacés par des aimants orientés afin d'augmenter l'attraction magnétique.

Avantageusement, il suffit donc que l'opérateur approche le support de l'écarteur pour qu'ils se solidarisent l'un à l'autre. L'utilisation d'une attache magnétique permet ainsi une fixation simple, fiable et très précise. L'utilisation d'une attache magnétique permet aussi, de manière particulièrement avantageuse, de fixer l'écarteur sur le support « en aveugle », ce qui rend cette fixation possible sans formation particulière.

De préférence encore, les attaches du support à l'écarteur sont conformées pour assurer un attachement dans une position prédéterminée du support par rapport à l'écarteur. En particulier, une partie d'attache peut présenter une forme conique qui, lors de l'opération d'attachement, pénètre dans un logement de la partie d'attache correspondante afin d'assurer son centrage par rapport audit logement.

De préférence, les parties d'attache de l'écarteur et du support sont conformées de manière que l'écarteur ne puisse être fixé sur le support que dans une unique position prédéterminée. Le risque d'erreur lors de l'assemblage de l'écarteur et du support est donc réduit.

Dans un mode de réalisation, comme représenté sur les figures 11A, 11B et 11C, une attache, de préférence chaque attache comporte une partie d'attache d'écarteur 27a' et 27b' mobile en translation par rapport au support, suivant un axe de translation Ta et Tb, respectivement.

Dans le mode de réalisation de la figure 11C, chaque partie d'attache de support 27a et 27b comporte un manchon 29a et 29b, respectivement, monté mobile en translation, suivant l'axe de translation Ta et Tb, respectivement, sur le corps du support.

Sur les figures 11A et 11C, les parties d'attache d'écarteur 27a' et 27b' sont en position de rapprochement maximal du support. Sur la figure 11B, elles sont en position d'écartement maximal du support. L'amplitude de la translation de la partie d'attache d'écarteur par rapport à la partie d'attache de support est de préférence supérieure à 0,5 cm et inférieure à 2 cm, de préférence inférieure à 1 cm.

Les axes de translation Ta et Tb sont de préférence symétriques par rapport au plan longitudinal médian P₁₂ du support. De préférence, chaque axe de translation Ta et Tb forme, avec le plan longitudinal médian P₁₂ du support, un angle inférieur à 30°, de préférence inférieur à 20°, et/ou, de préférence supérieur à 5°, de préférence supérieur à 10°. De préférence, un plan perpendiculaire à l'axe Ta ou Tb forme avec un plan perpendiculaire à l'axe X de l'ouverture d'écarteur un angle inférieur à 30°, de préférence inférieur à 20°, et/ou, de préférence supérieur à 5°, de préférence supérieur à 10°.

Dans la position de service, chaque partie d'attache d'écarteur peut donc être écartée du support en tirant l'écarteur vers l'avant.

De préférence encore, l'attache comporte un organe de rappel de la partie d'attache du support vers la partie d'attache de l'écarteur, par exemple un ressort de rappel élastique ou un aimant, de préférence un aimant. A défaut d'action par l'opérateur, les deux parties d'attache sont de préférence plaquées l'une contre l'autre.

De préférence, la partie d'attache d'écarteur est mobile en rotation et en translation par rapport à la partie d'attache de support. De préférence, l'orientation de l'axe de translation Ta - Tb n'est pas modifiée par ladite rotation.

De préférence, les moyens pour fixer l'écarteur au support sont configurés pour que, dans la position de service, l'écarteur soit élastiquement fléchi autour d'un axe V, de préférence sensiblement vertical. L'ergonomie en est améliorée.

De préférence, dans la position de service, l'angle formé par les deux plans dans lesquels s'étendent les deux parties de l'écarteur (22a, 22b), respectivement, est supérieur à 120°, de préférence supérieur à 140° et/ou inférieur à 170°, de préférence inférieur à 160°.

Dans un mode de réalisation, l'écarteur est configuré de manière à ce que l'angle formé par lesdits deux plans dans lesquels s'étendent les deux parties de l'écarteur (22a, 22b), respectivement, soit supérieur à 120°, de préférence supérieur à 140°, et/ou inférieur à 170°, de préférence inférieur à 160°, lorsque ledit écarteur est au repos, c'est-à-dire avant d'être positionné dans la position de service.

De préférence (Fig. 4D et 6), la distance *d_{27'}* entre les deux parties d'attache d'écarteur est inférieure à la distance *d₂₇* entre les deux parties d'attache de support correspondantes, de sorte que l'écarteur, lorsqu'il est fixé sur les paliers du support, soit fléchi. De préférence, la distance entre les deux parties d'attache d'écarteur est inférieure de plus de 2%, plus de 5% à la distance entre les deux parties d'attache de support.

### Téléphone mobile

Le téléphone portable 16 est un appareil personnel, conventionnellement de forme sensiblement parallélépipédique, comportant classiquement un appareil photo.

L'appareil photo du téléphone portable fournit de préférence des photos en couleurs, et/ou des photos infrarouges. Les photos infrarouges permettent avantageusement de faire apparaître les dents avec un excellent contraste.

Dans un mode de réalisation préféré, le téléphone portable comporte un applicatif permettant de guider l'opérateur lors des opérations, par exemple pour lui indiquer comment fixer l'écarteur ou le téléphone portable, ou l'orientation à donner au support pour prendre des photos particulières.

### Support

Le support 12 du kit représenté sur les figures 1A-1B, 2A-2B, 3A-3B, et 4A-4D comporte un boîtier 30, une porte 32, ou « capot », et un crochet de verrouillage 34.

De préférence, le support porte un miroir fixé, de préférence à l'intérieur du boîtier 30, et orienté de manière que, dans la position de service, l'appareil photo du téléphone portable reçoive une image composée comportant une image directe de l'ouverture d'écarteur et une image de l'ouverture d'écarteur réfléchie par le miroir (Fig. 8). Il acquiert ainsi simultanément plusieurs images des dents, observées selon des angles différents. La procédure d'acquisition de photos en est accélérée.

De préférence encore, le support comporte, de préférence à l'intérieur du boîtier 30,
- une mire colorimétrique et/ou une mire de translucidité, de préférence une mire colorimétrique et une mire de translucidité ; et
- de préférence une source lumineuse orientée de manière à éclairer d'une part les dents du patient à travers l'ouverture d'écarteur et d'autre part ladite mire colorimétrique et/ou ladite mire de translucidité,
le support étant configuré de manière que, dans la position de service, l'appareil photo reçoive une image de l'ouverture d'écarteur d'une part et de ladite mire colorimétrique et/ou de ladite mire de translucidité d'autre part. Avantageusement, les photos acquises permettent ainsi de déterminer avec précision la couleur et la translucidité des dents et/ou des tissus mous comme les gencives. En particulier, l'éclairage peut être contrôlé de manière à limiter les perturbations par l'environnement lumineux.

Dans le mode de réalisation représenté, le boîtier, d'axe longitudinal L (Fig. 4C), ne débouche vers l'extérieur que sur deux faces d'extrémité opposées, par une ouverture proximale 36, du côté du téléphone portable, et une ouverture distale 38, du côté de l'écarteur, respectivement (Fig. 1A, 1B, 3B). De préférence, les ouvertures proximale et distale sont sensiblement parallèles. De préférence, dans la position de service, elles sont sensiblement perpendiculaires à l'axe optique O de l'appareil photo.

De préférence, la longueur *L₃₀* du boîtier entre les ouvertures proximale et distale est inférieure à 20 cm, 15 cm, 10 cm, 7 cm ou 5cm, et/ou supérieure à 2 cm. Une longueur faible permet avantageusement d'acquérir des photos de régions de l'intérieur de la bouche proches de l'écarteur.

Autour de l'ouverture proximale 36, le boîtier définit une face de réception 40, de préférence plane, pour recevoir le téléphone portable 16 (Fig. 1A).

La porte 32 est montée mobile à rotation, sur le boîtier, autour d'un axe P, entre des positions ouverte et fermée. L'axe P est de préférence sensiblement horizontal et, dans la position de service, de préférence sensiblement parallèle à l'axe Y de rotation du support sur l'écarteur.

Le téléphone portable est fixé de manière amovible sur le support 12. La fixation est donc désactivable de manière réversible, c'est-à-dire que l'opérateur peut à volonté solidariser et désolidariser le téléphone portable du support. De préférence, la fixation du téléphone portable est assurée par compression contre la face de réception 40, au moyen de la porte 32 dans sa position fermée (Fig. 4A, 4B).

La face de réception 40 est de préférence revêtue d'une mousse améliorant le frottement avec le téléphone portable. De préférence, la mousse est compressible élastiquement, ce qui permet de serrer le téléphone portable contre la face de réception 40 sans l'endommager.

De préférence, la porte est verrouillée, dans la position fermée, au moyen du crochet de verrouillage 34. A cet effet, le crochet de verrouillage est monté mobile à rotation, sur le boîtier, autour d'un axe C, entre des positions verrouillée et déverrouillée dans lesquelles il s'oppose et ne s'oppose pas à l'ouverture de la porte 32, respectivement (Fig. 1A). L'axe C est de préférence sensiblement horizontal et de préférence sensiblement parallèle à l'axe Y de rotation du support sur l'écarteur.

Dans un mode de réalisation, le crochet de verrouillage et le boîtier comporte un aimant 44 et un insert métallique, respectivement, assurant le maintien du crochet de verrouillage dans la position verrouillée (Fig. 4A, 4B).

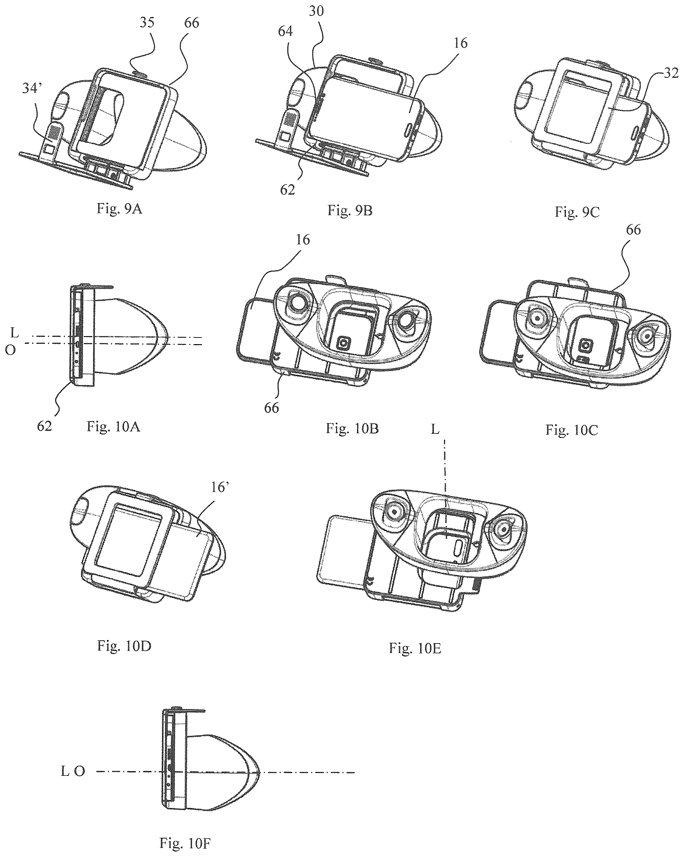

Les figures 9A-9C montrent un mode de réalisation dans lequel le crochet de verrouillage prend la forme d'une languette 34', de préférence élastique, percée d'un trou, et qui coopère avec un pion 35 fixé sur le boîtier. Après avoir positionné le téléphone portable (figure 9B), l'insertion du pion dans le trou de la languette maintient la porte dans la position fermée (figure 9C).

De préférence, la porte 32 comporte une fenêtre 46 à travers laquelle l'opérateur peut voir l'écran au dos du téléphone portable. La fenêtre peut être une découpe ou être constituée en un matériau transparent. Une découpe permet avantageusement à l'opérateur d'interagir avec l'écran tactile du téléphone portable.

La porte 32 comporte de préférence une plaque d'appui 48 sensiblement plane et un pied 49 faisant saillie du plan général de la plaque d'appui 48, portant un pivot « de porte » 50 définissant l'axe de rotation P de la porte 32 (Fig. 1A, 2A).

De préférence, au moins les parties de la plaque d'appui 48 qui s'étendent contre l'écran du téléphone portable sont transparentes. L'opérateur a ainsi une meilleure vision sur la photo qu'il s'apprête à prendre. De préférence toute la plaque d'appui 48 est transparente.

La face intérieure 52 de la plaque d'appui 48, qui fait face à la face de réception 40 dans la position fermée, appuie sur le téléphone portable dans la position fermée. De préférence, la face intérieure 52 porte des plots élastiques 54, de préférence en un élastomère, qui évitent le contact direct de la face intérieure 52 avec le téléphone portable, et limitent donc le risque d'endommager ce dernier ou la plaque d'appui 48 (Fig. 1A, 3B).

Le pivot de porte 50 est de préférence monté coulissant suivant un axe de coulissement Z perpendiculaire à l'axe de rotation P et, de préférence perpendiculaire au plan général de la face de réception 40.

Le pivot de porte 50 est de préférence monté coulissant dans un rail 56 ménagé sur le boîtier ou dans la porte, de préférence dans le pied (figure 3b), de manière que la distance d entre le pivot de porte 50 et le plan général de la plaque d'appui soit modifiable lorsque la plaque d'appui 48 s'étend parallèlement à la face de réception 40.

De manière équivalente, la porte peut comporter un rail d'axe Z dans lequel coulisse un pion du boîtier.

Avantageusement, des téléphones mobiles de différentes épaisseurs peuvent être fixés sur le support, sans risque d'endommagement. De préférence, un ressort est disposé dans le rail 56 de manière à pousser ou tirer élastiquement le pivot de porte 50 vers l'ouverture proximale du boîtier. Avantageusement, aucune manipulation spécifique n'est donc nécessaire pour que la position du pivot de porte 50 s'adapte à l'épaisseur du téléphone portable. En outre, avantageusement, le rappel élastique du pivot de porte assure, dans la position fermée, une compression du téléphone portable entre la plaque d'appui 48 et la face de réception 40.

Dans la position ouverte, la porte laisse la face de réception 40 accessible pour que l'opérateur y déposer ou extraire le téléphone portable.

De préférence, dans la position ouverte, lorsque la porte repose à plat sur un substrat S plan, de préférence par l'intermédiaire d'au moins une entretoise 58 fixée sur la plaque d'appui 48, le boîtier 30 prend appui, par au moins une zone d'appui 60, sur ledit substrat. De préférence, l'angle θ entre le plan de la face de réception 40 et le plan de la plaque d'appui est supérieur à 110°, 120°, voire 125° et/ou de préférence inférieur à 150°, 140°, voire 135°. Il est ainsi particulièrement aisé de déposer le téléphone portable sur la face de réception 40 ou de l'en extraire.

De préférence, le boîtier et/ou la porte 32 définissent une butée inférieure 62, disposée de manière que, dans la position ouverte, lorsque la porte repose à plat sur le substrat S, la butée inférieure 62 empêche un glissement du téléphone portable vers le bas.

De préférence, le boîtier et/ou la porte 32 définissent encore une butée latérale 64, de préférence une unique butée latérale 64, disposée de manière à limiter le glissement latéral du téléphone portable, c'est-à-dire vers la gauche dans le mode de réalisation représenté.

Lorsque le téléphone portable est en contact avec les butées inférieure et latérale, l'appareil photo observe l'intérieur du boîtier, et en particulier l'ouverture distale du boîtier. L'opérateur n'a donc aucune difficulté à placer correctement le téléphone portable.

De préférence, le support ne comporte pas d'autres butées. Le support peut ainsi servir à des téléphones mobiles de longueurs et/ou de largeurs variées.

Dans un mode de réalisation, comme représenté sur les figures 9A-9C et 10A-10F, le téléphone portable peut être déplacé par rapport au boîtier.

De préférence, il est fixé sur un cadre 66 qui définit une butée inférieure 62 et une butée latérale 64 assurant un positionnement prédéterminé du téléphone portable lorsqu'il est en contact avec lesdites butées.

L'axe optique O du téléphone portable peut être décalé de l'axe longitudinal L du support, comme représenté sur la figure 10A. La mobilité du cadre 66 permet avantageusement d'aligner ces deux axes.

Dans un mode de réalisation, le cadre 66 n'est mobile que verticalement, comme représenté sur les figures 10B et 10C. De préférence, le cadre comporte un coulisseau qui est monté coulissant dans un rail du boîtier.

Dans un mode de réalisation, le cadre 66 n'est mobile que verticalement et horizontalement, ce qui permet de positionner le téléphone portable dans une position quelconque dans le plan du cadre.

Lorsqu'un autre téléphone portable 16' est utilisé (Figure 10D-10F), la position du cadre peut être avantageusement ajustée pour confondre l'axe optique O et l'axe longitudinal L.

Pour que cet ajustement soit rapide, le cadre comporte de préférence des marques d'identification et le boîtier comporte de préférence un repère. Un alignement d'une marque d'identification sur le repère permet avantageusement de connaître précisément la position du cadre par rapport au boîtier. Bien entendu, de manière équivalent, les marques d'identification peuvent être portées par le boîtier 30 et le repère être porté par le cadre 66.

De préférence, les marques d'identification sont déterminées de manière que l'alignement d'une marque avec le repère indique un alignement de l'axe optique de tout téléphone portable d'un modèle particulier avec l'axe longitudinal du boîtier. Autrement dit, à chaque modèle de téléphone correspond une marque disposée de manière que lorsqu'elle est en regard du repère, un téléphone de ce modèle fixé sur le cadre est correctement positionné sur le support.

### Fonctionnement

Le fonctionnement du kit découle directement de la description qui précède.

Initialement, l'opérateur ouvre la porte 32 du support. Il peut, pour plus de stabilité, poser le support sur un substrat, par exemple une table (figures 1A et 1B).

L'opérateur dépose le téléphone portable sur la face de réception 40, de manière qu'il soit en butée sur la butée latérale 64 et sur la butée inférieure 62 (figures 2A et 2B).

Le téléphone portable obture alors l'ouverture proximale et l'axe optique O de son appareil photo passe par l'ouverture distale, ce qui lui permet de prendre des photos à travers l'ouverture distale. Après fixation de l'écarteur, le téléphone portable peut ainsi prendre des photos à travers l'ouverture d'écarteur.

Puis l'opérateur ferme la porte 32.

Après que la porte 32 est entrée en contact avec le téléphone portable, la poursuite de sa fermeture vient tirer sur le pivot de porte 50 à l'encontre du ressort logé dans le rail 52. Lorsque la porte atteint la position fermée, la mousse 42 et les plots 54 sont comprimés élastiquement. Ils prennent en étau, et donc bloquent en position, le téléphone portable (figure 3A et 3B). La porte peut également se déformer élastiquement (figure 4C) et ainsi participer au serrage du téléphone portable. Cependant, de préférence, la traction sur le pivot de porte 50 est adaptée de manière que, dans la position fermée, la porte reste sensiblement plane.

Ensuite, l'opérateur rabat le crochet de verrouillage pour immobiliser la porte en position fermée (figure 4A, 4B, 4C et 4D). Le crochet de verrouillage est maintenu dans la position verrouillée par crochetage du boîtier et/ou action de l'aimant 44.

L'opérateur voit l'écran tactile à travers la fenêtre 46 et, de préférence, a accès à l'écran tactile du téléphone portable, ce qui lui permet, notamment, de contrôler l'appareil photo.

L'opérateur fixe également l'écarteur sur le support. Cette opération peut être antérieure ou postérieure à la fixation du téléphone portable.

A cet effet, l'opérateur approche l'écarteur du support pour disposer les parties de l'attache d'écarteur en face des parties d'attache de support correspondantes.

L'attraction magnétique conduit à l'activation de l'attache de manière rapide, précise et sans précaution particulière. Lors de cette activation, le guidage et/ou la forme des parties de l'attache assurent un positionnement prédéterminé de l'écarteur par rapport au support.

L'écarteur ceinture alors, au moins partiellement, le centre de l'ouverture distale, de sorte que l'ouverture d'écarteur et l'ouverture distale sont, au moins en partie superposées.

Le kit est alors prêt à être utilisé (figures 5A et 5B).

Dans cette position, le support est mobile en rotation autour de l'écarteur, autour de l'axe Y.

Le patient, qui peut être également l'opérateur, dispose alors ses lèvres dans les goulottes définies par le rebord 22 de l'écarteur (figure 7). L'écarteur est classiquement configuré de manière à écarter élastiquement l'une de l'autre les lèvres supérieure et inférieure du patient de manière à dégager les dents et les rendre visibles à travers l'ouverture d'écarteur. En particulier, à la différence d'un écarteur de mâchoires, l'écarteur n'appuie pas sur les dents de manière à écarter les deux mâchoires l'une de l'autre. L'écarteur permet donc de retrousser les lèvres. L'écarteur permet ainsi d'observer les dents sans être gêné par les lèvres. La mobilité relative des deux parties 22a et 22b du rebord et l'élasticité de la poignée 25 facilite cette opération.

Les dents du patient sont alors bien dégagées, notamment grâce aux oreilles 26a et 26b. Le boîtier fermé masque avantageusement les dents, ce qui préserve l'intimité du patient. L'appareil photo du téléphone portable voit une image, voire, si le support comporte un miroir, une image composée I_{c} comportant une image directe I_{d} et une ou plusieurs images réfléchies Iᵣ par le ou les miroirs (figure 8).

L'écarteur n'interdit pas le rapprochement ou l'écartement des mâchoires l'une de l'autre.

Il autorise ainsi l'acquisition de photos, représentant les dents du patient, alors que le patient a la bouche ouverte ou la bouche fermée, en conservant l'écartement des lèvres.

En appuyant sur le déclencheur de l'appareil d'acquisition, l'opérateur acquiert une ou plusieurs photos.

En faisant pivoter le support autour de l'axe Y, l'appareil photo peut observer des régions différentes de la bouche. En particulier, lorsque l'axe Y de pivotement est horizontal, il permet de prendre des photos de la voute dentaire dans des conditions déterminées.

De préférence, le segment de l'axe Y qui s'étend entre les deux attaches du support (27a, 27b), s'étend entre les deux plans horizontaux qui passent par les extrémités inférieure et supérieure de l'ouverture d'écarteur respectivement.

De préférence, il s'étend à moins de 5 cm, 2 cm, 1 cm du plan horizontal qui passe par l'axe optique O et/ou qui passe par l'axe longitudinal L.

De préférence, ledit segment est écarté de moins de 10 cm, 5 cm, 3 cm, 2 cm de l'ouverture distale.

De préférence, l'axe de pivotement du support par rapport à l'écarteur est sensiblement dans un plan perpendiculaire à l'axe longitudinal L et/ou à l'axe optique O, et de préférence horizontal. Notamment, la distance entre l'appareil photo et l'ouverture d'écarteur est de préférence sensiblement constante.

De préférence, la distance entre l'appareil photo et l'ouverture d'écarteur est inférieure à 20 cm, 15 cm, 10 cm, 7 cm ou 5 cm, et/ou supérieure à 2 cm.

Une distance faible permet avantageusement d'acquérir des photos de régions de l'intérieur de la bouche proches de l'écarteur.

Cela permet avantageusement d'augmenter la précision de la prise des photos lors de la manipulation.

De plus, le réglage de l'appareil photo et l'analyse ultérieure des photos acquises en sont facilités.

La possibilité de faire translater d'une partie d'attache d'écarteur suivant l'axe Ta ou Tb permet de prendre des photos latérales de manière confortable, sans déformation excessive de la bouche.

Dans un mode de réalisation, la prise de plusieurs photos successives résulte d'un unique actionnement du déclencheur de l'appareil photo. En particulier, l'appareil photo peut être configuré pour prendre successivement plusieurs photos, avec des distances focales différentes, en conséquence d'un unique déclenchement.

Par ailleurs, le cas échéant, l'appareil photo acquiert une photo des dents du patient représentant également les mires colorimétriques et de translucidité, ce qui permet une correction des couleurs de la photo. Pour que la correction soit précise, la source lumineuse est de préférence réglée de manière que les mires soient éclairées dans les mêmes conditions que lors de la mesure des propriétés réelles. L'utilisation d'un boîtier fermé facilite le contrôle de l'éclairage.

Comme cela apparaît clairement à présent, l'invention facilite considérablement l'acquisition de photos, y compris de photos représentant les faces vestibulaires de molaires, en particulier pour la mise en oeuvre du procédé décrit dans PCT/EP2015/074896. Elle permet une acquisition très rapide de plusieurs photos, typiquement en moins d'une minute, sans avoir recours à une personne spécialisée, notamment un dentiste ou un orthodontiste. L'acquisition de photos peut être en particulier effectuée par le patient lui-même ou par un de ses proches, avec un simple téléphone portable, n'importe où, et en particulier en dehors d'un cabinet médical, dentaire ou d'orthodontie. En outre, l'acquisition est possible sans utilisation d'un outil prenant appui sur le sol pour immobiliser le téléphone portable, et notamment sans trépied.

En outre, elle permet une fixation rapide et fiable du téléphone portable, quelle que soit son épaisseur, sans risque de l'endommager.

Enfin, la mobilité guidée de l'écarteur par rapport au téléphone portable permet de facilement prendre des photos de régions de la bouche difficile d'accès.

Bien entendu, l'invention n'est pas limitée aux modes de réalisation décrits et représentés, fournis à des fins illustratives seulement.

Notamment, la forme du boîtier n'est pas limitative.

L'articulation n'est pas limitée à une liaison pivot. Dans un mode de réalisation particulier, le support est monté à rotule sur l'écarteur.

De préférence, le centre de la rotation du support par rapport à l'écarteur est compris dans un plan horizontal qui s'étend entre deux plans horizontaux qui passent par les extrémités supérieure et inférieure de l'ouverture d'écarteur respectivement. De préférence, le centre de la rotation du support par rapport à l'écarteur est compris dans un plan qui s'étend à moins de 5 cm, 2 cm, 1 cm du plan horizontal qui passe par l'axe optique O, et/ou qui passe par l'axe longitudinal L.

De préférence, le centre de la rotation du support par rapport à l'écarteur est situé sur l'axe optique et/ou sur l'axe longitudinal.

Cela permet une meilleure précision de la prise des photos lors de la manipulation.

Le support peut être fixé à l'écarteur par tout moyen, par exemple au moyen de clips, et/ou de bandes auto-agrippantes de type Velcro^{®}, et/ou de mâchoires de serrage, et/ou de vis, et/ou d'aimants, et/ou par complémentarité de forme entre le support et l'écarteur.

Le téléphone portable peut être fixé sur le support par tout moyen, par exemple choisis dans le groupe constitué par des moyens de clipsage, des bandes auto-agrippantes de type Velcro^{®}, des mâchoires de serrage, des vis, des aimants, et une complémentarité de forme entre le support et le téléphone portable.

L'appareil photo n'est pas limité à l'appareil photo d'un téléphone portable. Tout autre appareil personnel couramment disponible dans le commerce, comportant un appareil photo peut être utilisé, par exemple un appareil photo dit « connecté », une montre dite « intelligente », ou « smartwatch », ou une tablette ou un ordinateur personnel, fixe ou portable, comportant un système d'acquisition d'images, comme une webcam ou un appareil photo, de préférence un appareil photo numérique. Il pèse de préférence moins de 3 kg, moins de 2 kg, moins de 1 kg, moins de 500 g, de préférence moins de 300 g.

## Revendications

1. Kit de prise de photos dentaires comportant :
- un support (12) comportant une partie d'attache de support (27a;27b) ;
- un écarteur dentaire (14) définissant une ouverture d'écarteur (24) et comportant une partie d'attache d'écarteur (27a';27b') fixée de manière amovible, dans une position montée de l'écarteur sur le support, sur la partie d'attache de support (27a;27b) ; et
- un appareil photo (16) fixé, de manière amovible, sur le support dans une position de service dans laquelle l'appareil photo est orienté pour recevoir une image au moins partielle de l'ouverture d'écarteur,
**caractérisé en ce que** l'appareil photo, d'axe optique O, est intégré dans un téléphone portable et le téléphone portable est monté mobile sur le support dans un plan perpendiculaire audit axe optique.

2. Kit selon la revendication 1, le support (12) comportant un boîtier et un cadre de fixation du téléphone portable, le cadre étant guidé en translation sur le boîtier.

3. Kit selon la revendication 2, le cadre ou le boîtier comportant des marques d'identification d'une position du cadre par rapport au boîtier ou du boîtier par rapport au cadre respectivement, le boîtier, ou respectivement le cadre, comportant un repère en face duquel chaque marque d'identification peut être positionnée, de préférence les marques d'identification correspondant à des modèles de téléphone portable.

4. Kit selon l'une quelconque des revendications 2 et 3, le boîtier comportant un moyen, sélectivement activable et désactivable par un opérateur, pour bloquer le téléphone portable en position.

5. Kit selon l'une quelconque des revendications précédentes, la mobilité du téléphone portable étant déterminées par des positions stables prédéterminées.

6. Kit selon l'une quelconque des revendications précédentes, la mobilité du téléphone portable étant limitée à une translation suivant un axe ou à deux translations suivant deux axes, de préférence sensiblement perpendiculaires l'un à l'autre.

7. Kit selon l'une quelconque des revendications précédentes, dans lequel la partie d'attache d'écarteur est articulée sur la partie d'attache de support.

8. Kit selon l'une quelconque des revendications précédentes, dans lequel au moins une, de préférence chaque attache de l'écarteur sur le support, autorise une rotation et un écartement de la partie d'attache d'écarteur par rapport au support.

9. Kit selon la revendication 8, dans lequel la partie d'attache d'écarteur (27a';27b') est montée pivotante sur la partie d'attache de support (27a;27b) autour d'un axe de pivotement (Ya;Yb) qui, dans la position de service, s'étend horizontalement.

10. Kit selon l'une quelconque des deux revendications 8 et 9, dans lequel l'axe de pivotement est mobile en translation par rapport au support, suivant une direction de translation (Ta;Tb) différente de celle de l'axe de pivotement (Ya;Yb).

11. Kit selon l'une quelconque des revendications 8 à 10, dans lequel l'amplitude maximale de pivotement (α) du support par rapport à l'écarteur est supérieure à 5° et inférieure à 30°.

12. Kit selon l'une quelconque des revendications précédentes, dans lequel la partie d'attache d'écarteur est fixée magnétiquement sur la partie d'attache du support.

13. Kit selon la revendication 12, dans lequel l'écarteur porte deux parties d'attache d'écarteur (27a';27b') fixées de manière amovible sur deux parties d'attache de support (27a;27b), respectivement, la distance entre les deux parties d'attache d'écarteur (*d_{27'}*) étant inférieure à la distance entre les deux parties d'attache de support (*d₂₇*), de sorte que l'écarteur est fléchi.

14. Kit selon l'une quelconque des revendications précédentes, dans lequel le support comporte
- un corps sous la forme d'un boîtier (30), et
- une porte (32) montée pivotante sur le corps autour d'un pivot de porte (50) d'axe P, entre une position fermée dans laquelle elle maintient un téléphone portable serré contre une face de réception (40) du corps, et une position ouverte dans laquelle elle autorise une extraction du téléphone portable,
le pivot de porte étant monté coulissant en translation, selon un axe de coulissement (Z) non parallèle à l'axe P, par rapport au corps du support.

15. Kit selon une quelconque des revendications précédentes, dans lequel la distance entre l'appareil photo (16) et l'ouverture d'écarteur (24) est sensiblement constante, de préférence inférieure à 20 cm, 15 cm, 10 cm, 7 cm, ou 5 cm et/ou supérieure à 2 cm.
